# EUROPEAN PATENT APPLICATION

(11) **EP 4 686 469 A1**
(43) Date of publication of application: **04.02.2026**
(21) Application number: 24191979.4
(22) Date of filing: 31.07.2024
(51) Int. Cl.: A61K 31/10, A61K 31/341, A61K 31/41, A61K 31/415, A61K 31/4168, A61K 31/44, A61K 31/496, A61K 31/506, A61K 31/54, A61K 31/5513, A61K 31/64, A61K 31/69, A61K 38/00, A61K 39/00

(54) **CXCL8 INHIBITORS FOR USE IN THE TREATMENT OF DIABETES-ASSOCIATED COMORBIDITIES**

(71) Applicant: Dompé farmaceutici SpA, 20122 Milano (IT)
(72) Inventor: BRANDOLINI, Laura, 67100 L'Aquila (IT); BOCCELLA, Serena, 80131 Napoli (IT); GIORGIO, Cristina, 67100 L'Aquila (IT); LUONGO, Livio, 80138 Napoli (IT)
(74) Representative: Dompé farmaceutici Spa

(57) **Abstract**

The invention relates to CXCL8 inhibitors for use in the treatment of diabetes-associated comorbidities selected from diabetic retinopathy and/or diabetic neuropathy.

## Description

### TECHNICAL FIELD

The invention relates to CXCL8 inhibitors for use in the treatment of diabetes-associated comorbidities selected from diabetic retinopathy and/or diabetic neuropathy.

### BACKGROUND OF THE INVENTION

Diabetes mellitus (DM) is one of the biggest healthcare problems in the world, and it strongly associated with microvascular and macrovascular complications, typically also resulting in diabetes-associated comorbidities, including coexisting diabetic retinopathy (DR) and diabetic peripheral neuropathy (DPN).

Diabetic peripheral neuropathy is a common complication of diabetes with variable clinical presentations; in particular, it can be either with painful or painless symptoms.

Painful diabetic peripheral neuropathy (PDPN) affects 25-50% of patients manifesting neuropathic pain by mechanical allodynia (MA), which is one of the main reasons for patients to seek medical advice as it severely debilitates patients' life quality, as well as thermal hypersensitivity (Navarro X, Kennedy WR. Evaluation of thermal and pain sensitivity in type I diabetic patients. J Neurol Neurosurg Psychiatry. 1991 Jan;54(1):60-4; Raputova J, et al., Sensory phenotype and risk factors for painful diabetic neuropathy: a cross-sectional observational study. Pain. 2017 Dec;158(12):2340-2353). The pathogenesis of PDPN is complex. Diabetes can lead to multiple damage to the peripheral nervous system, including abnormal activation of multiple metabolic pathways and imbalance of mitochondrial redox state; in addition, nerve swelling caused by metabolic disturbances can result in nerve compression injury (Zhou H. et al., The Development of Mechanical Allodynia in Diabetic Rats Revealed by Single-Cell RNA-Seq. Front Mol Neurosci. 2022 May 20;15:856299).

Pharmacologic and non-pharmacologic interventions are available for the treatment of PDPN; however, there are few high-quality, and since the available studies use varying methodologies, it is difficult to know which treatment strategy may be most effective (Snyder MJ., et al., Treating Painful Diabetic Peripheral Neuropathy: An Update. Am Fam Physician. 2016 Aug 1 ;94(3):227-34).

Moreover, the main current pharmacological approaches (e.g. anticonvulsants and antidepressants) target relief of painful DPN symptoms, whereas a more pathogenetically oriented therapy should be preferred. Diabetic retinopathy (DR) is a microangiopathy of the retina, which involves changes in the vascular wall and in the rheological properties of the blood, thus resulting in capillary occlusion, retinal ischemia and angiographically demonstrable leakage.

The main types of diabetic retinopathy are non-proliferative diabetic retinopathy (NPDR), which is the early stage of the diabetic eye disease, and proliferative diabetic retinopathy (PDR), which is the more advanced stage of the diabetic eye disease (Kollias AN., et al., Diabetic retinopathy: Early diagnosis and effective treatment. Dtsch Arztebl Int. 2010 Feb;107(5):75-83).

In particular, the non-proliferative diabetic retinopathy typically includes formation of microaneurysms of the capillary wall, as well as blot hemorrhages, hard exudates, fluctuations of venous caliber ("venous beading"), and intraretinal microvascular anomalies (namely, dilated telangiectatic capillaries - in the area adjacent to capillary occlusions - considered a classic sign of ischemia and a predictor of imminent progression to proliferative retinopathy).

As hypoperfusion in the retinal capillary bed becomes more severe and spreads across the retinal area, proliferative diabetic retinopathy develops. As a reaction to ischemia, neovascularization arises at the papilla and on the retina outside the papilla, leading to epiretinal and subhyaloid vitreous hemorrhages and promoting the formation of pathological structures, the contraction of which leads, later on, to tractional retinal detachment or tractional macular edema, thus causing blindness.

The ultimate and most severe complication of diabetic retinopathy is neovascular glaucoma, wherein the newly formed vessels grow from the pupil into the chamber angle, obstructing the outflow of the aqueous humor, thus leading to painful blindness and shrinking of the eye.

Multiple biochemical signal pathways are involved in diabetic retinopathy, both proliferative and non-proliferative.

In particular, vascular endothelial growth factor (VEGF) is a mediator of vascular leakage, and is thus partly responsible for the collapse of the inner blood retinal barrier (Kollias AN., et al., Diabetic retinopathy: Early diagnosis and effective treatment. Dtsch Arztebl Int. 2010 Feb;107(5):75-83).

Besides, chronic low-grade inflammation is intimately correlated with the pathogenesis of DR, which is mainly caused by the activation of the innate immune system.

As well-known in the art, neutrophil extracellular traps (NETs), which are typically realsed during NETosis (a neutrophil-specific cell death process) may lead to diabetes, if excessive formation thereof occurs; in fact, circulating NETosis markers, for example myeloperoxidase (MPO) and post-translational modificated histones, including citrullined histone H3, are particularly increased in DR patients (Wang L, et al., Hyperglycemia Induces Neutrophil Extracellular Traps Formation Through an NADPH Oxidase-Dependent Pathway in Diabetic Retinopathy. Front Immunol. 2019 Jan 8;9:3076; Yang ML, Sodré FMC, Mamula MJ, Overbergh L. Citrullination and PAD Enzyme Biology in Type 1 Diabetes - Regulators of Inflammation, Autoimmunity, and Pathology. Front Immunol. 2021 ;12:678953). The main treatment strategies of diabetic retinopathies include laser photocoagulation, surgery (for example, pars plana vitrectomy (PPV)), and pharmacotherapy, including intravitreous glucocorticoids, VEGF antagonists, and anti-VEGF antibodies administration.

Different CXCL8 inhibitors have been developed and are well known to the skilled person.

WO2000/024710 discloses N-(2-aryl-propionyl)-sulfonamides, having inhibitory activity of neutrophils chemotaxis and degranulation induced by interleukin-8, and their use in the prevention and treatment of tissue damage due to the exacerbate recruitment of polymorphonuclear neutrophils (leukocytes PMN) at the inflammatory sites, in particular in the treatment of psoriasis, rheumatoid arthritis, ulcerative colitis, acute respiratory insufficiency, idiopathic fibrosis, glomerulonephritis.

WO2005/090295 discloses (R)-2-[4-(trifluoromethanesulfonyloxy)phenyl]propionic acid derivatives, which are used as inhibitors of the chemotaxis of polymorphonucleate and mononucleate cells, particularly in the treatment of neutrophils-dependent pathologies. The use of said compounds in the treatment of psoriasis, ulcerative colitis, melanoma, angiogenesis, chronic obstructive pulmonary disease (COPD), bullous pemphigo, rheumatoid arthritis, idiopathic fibrosis, glomerulonephritis and in the prevention and treatment of damages caused by ischemia and reperfusion is also disclosed.

WO2010/031835 discloses 2-aryl-propionic acids and derivatives, substituted in position 4 with 2-amino-heterocycles, as CXCL8-induced chemotaxis inhibitors, useful in the prevention and treatment of tissue damage due to the exacerbated recruitment of polymorphonucleated neutrophils (PMN leukocytes) at inflammation sites. The use of said compounds in the treatment of transient cerebral ischemia, damages caused by ischemia and reperfusion, bullous pemphigo, rheumatoid arthritis, idiopathic fibrosis and glomerulonephritis is also disclosed.

WO2011/042465 discloses the use of R(-)-2-[(4-isobutylphenyl)propionyl]-methanesulfonamide (also known as Reparixin) or R(-)-2-[(4'-trifluoromethanesulfonyloxy)phenyl]propionyl-methanesulfonamide (also known as Meraxin or Ladarixin) for the prevention of Type 1 diabetes, by delaying the onset and the progression of diabetes, as well as maintaining constantly and significantly low the glycaemic levels compared to vehicle-treated mice.

Ladarixin is effective in treating diabetes, in particular type 1 diabetes, by selectively inhibiting pancreatic inflammation and preserving residual-cell function (Castelli V, et al., CXCR1/2 Inhibitor Ladarixin Ameliorates the Insulin Resistance of 3T3-L1 Adipocytes by Inhibiting Inflammation and Improving Insulin Signaling. Cells. 2021 Sep 6;10(9):2324).

The present invention is aimed at providing an effective treatment of diabetes-associated comorbidities, in particular diabetic retinopathy and/or diabetic neuropathy.

### SUMMARY OF THE INVENTION

The invention is directed to a CXCL8 inhibitor for use in the treatment of diabetes-associated comorbidities selected from diabetic retinopathy and/or diabetic neuropathy.

The invention is also directed to a pharmaceutical composition comprising at least one CXCL8 inhibitor and at least one pharmaceutically acceptable excipient or carrier, for use in the treatment of diabetes-associated comorbidities selected from diabetic retinopathy and/or diabetic neuropathy.

The invention is also directed to a method of preventing and/or treating diabetes-associated comorbidities selected from diabetic retinopathy and/or diabetic neuropathy, in a subject in need thereof.

The invention is also directed to the use of a CXCL8 inhibitor in the manufacture of a medicament for preventing and/or treating diabetes-associated comorbidities selected from diabetic retinopathy and/or diabetic neuropathy.

### BRIEF DESCRIPTION OF FIGURES

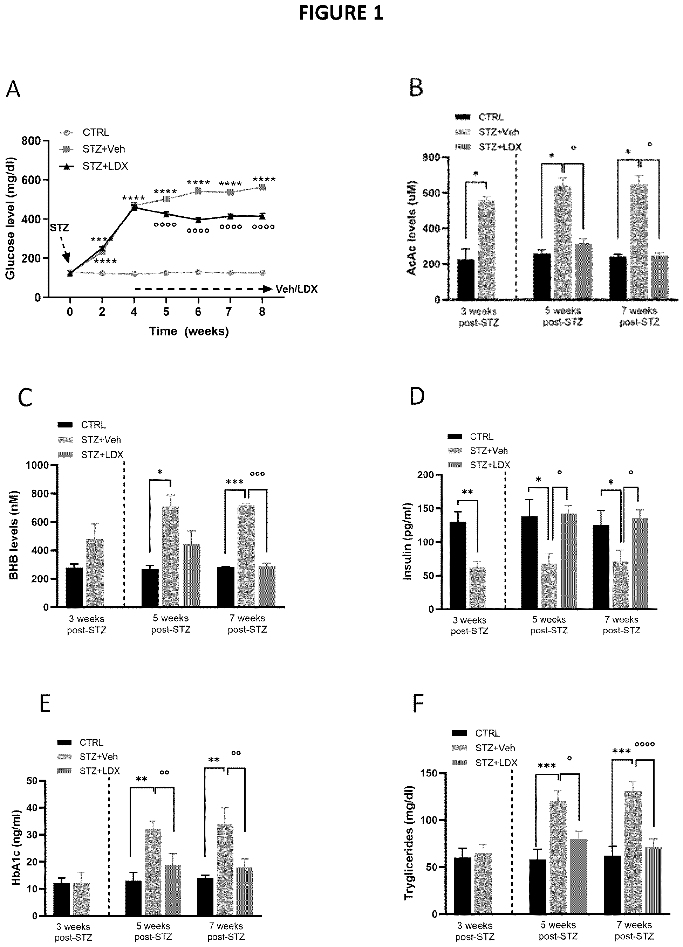

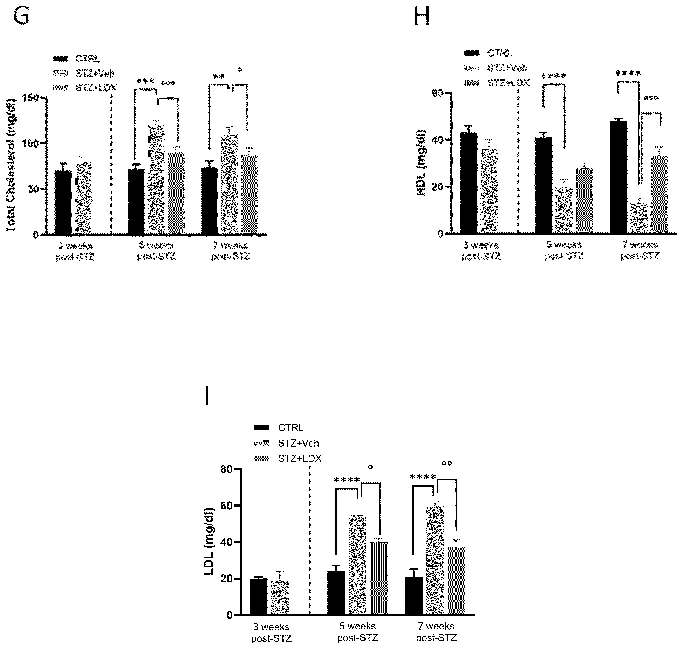

**Figure 1** shows graphs of (A) Glucose level (mg/dl), (B) AcAc (µM), (C) BHB (nM), (D) Insulin (pg/ml), (E) HbA1c (ng/ml), (F) Triglycerides (mg/dl), (G) Total Cholesterol (mg/dl), (H) HDL (mg/dl) and (I) LDL (mg/dl) of CTRL, STZ+Veh and STZ+LDX animals (early treatment), according to Example 1. Data are represented as mean ± SEM (N= 4). Two-way ANOVA followed by Tukey's post hoc tests for multiple comparisons tests were performed among groups during the time course. The threshold of 0.05 was used for determining statistical significance. * and ° indicate significant differences vs CTRL and STZ+Veh, respectively.

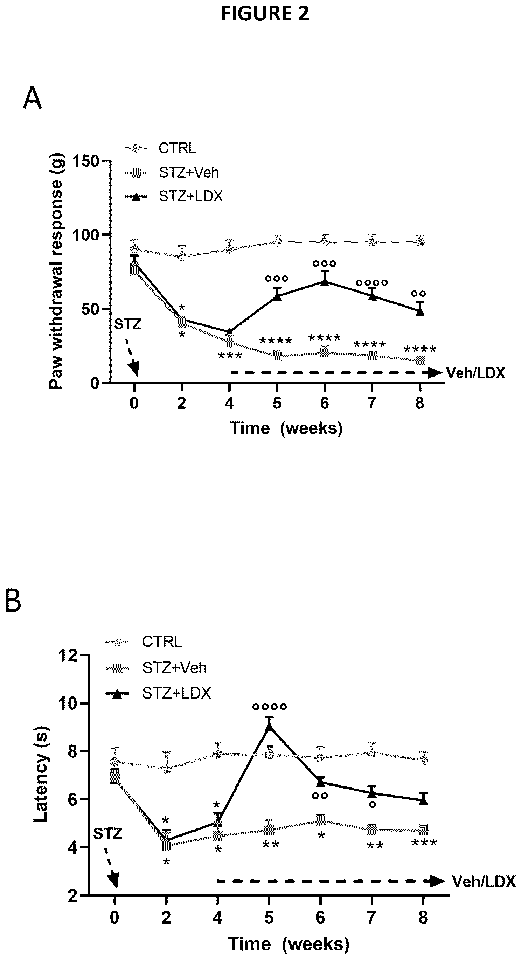

**Figure 2** shows graphs of (A) Paw withdrawal response (g), and (B) Latency (s) of CTRL, STZ+Veh and STZ+LDX animals (early treatment), according to Example 1. Data are represented as mean ± SEM (N= 3). Two-way ANOVA followed by Tukey's post hoc tests for multiple comparisons tests were performed among groups during the time course. The threshold of 0.05 was used for determining statistical significance. * and ° indicate significant differences vs CTRL and STZ+Veh, respectively.

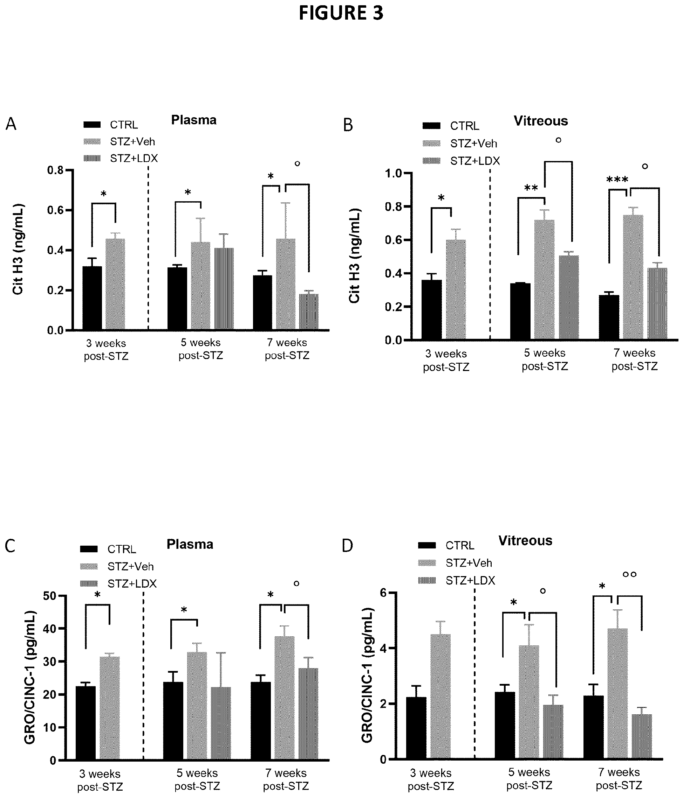

**Figure 3** shows graphs of Citrullinated Histone H3 (Cit H3) concentration (ng/mL) in plasma (A) and vitreous (B), and GRO/CINC-1 concentration (CXCL8) (pg/mL) in plasma (C) and vitreous (D), in CTRL, STZ+Veh and STZ+LDX animals (early treatment), according to Example 1. Data are represented as mean ± SEM (N= 4). Two-way ANOVA followed by Tukey's post hoc tests for multiple comparisons tests were performed among groups during the time course. The threshold of 0.05 was used for determining statistical significance. * and ° indicate significant differences vs CTRL and STZ+Veh, respectively.

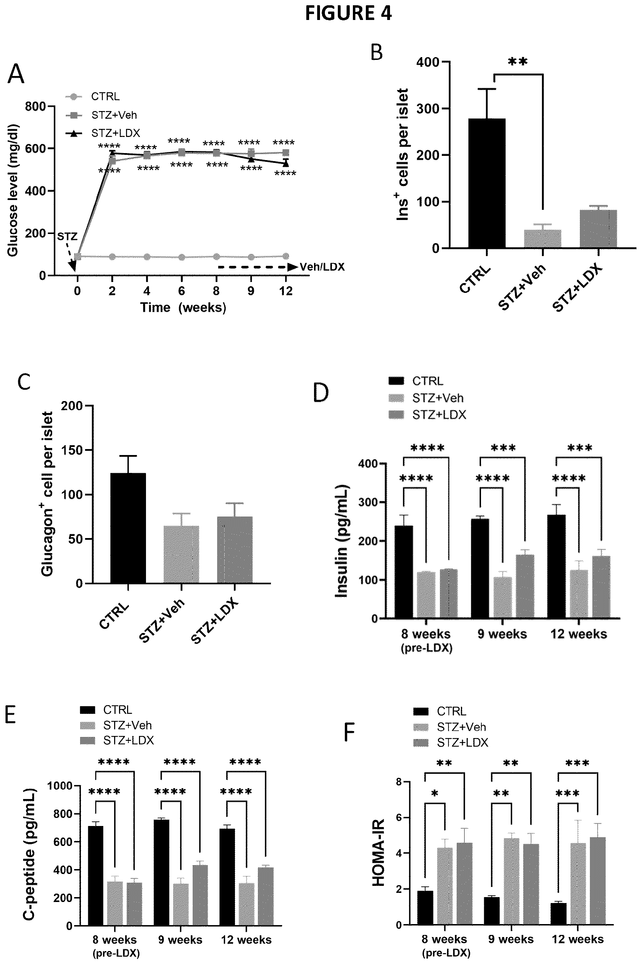

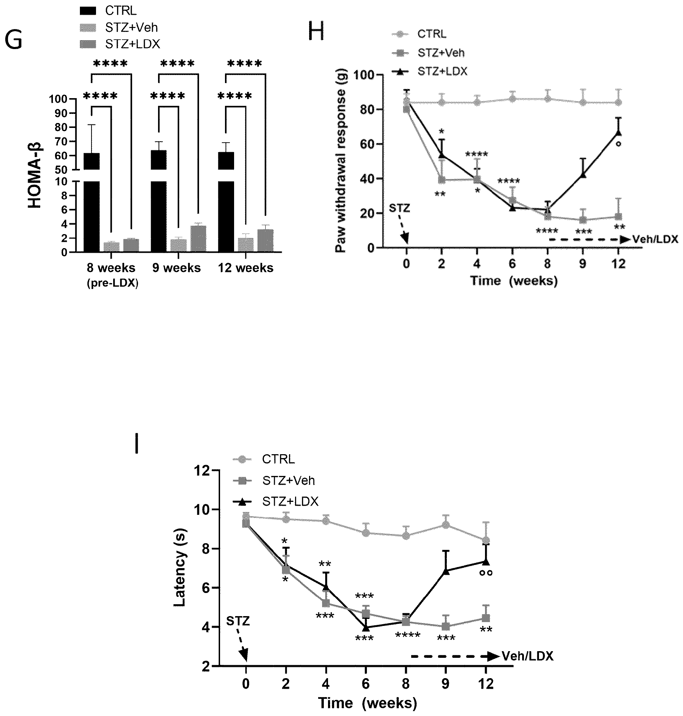

**Figure 4** shows graphs of (A) glucose level (mg/dl), mean of insulin (Ins⁺) (B) and glucagon (Glucagon⁺) (C) positive cells per islet, (D) plasmatic insulin (pg/ml), (E) plasmatic C-peptide (pg/ml), (F) Homa-IR, (G) HOMA-β, (H) paw withdrawal threshold (g), and (I) latency (s), in CTRL, STZ+Veh and STZ+LDX animals (late treatment), according to Example 1. Data are represented as mean ± SEM (N= 4). Two-way ANOVA followed by Tukey's post hoc tests for multiple comparisons tests were performed among groups during the time course. The threshold of 0.05 was used for determining statistical significance. * and ° indicate significant differences vs CTRL and STZ+Veh, respectively.

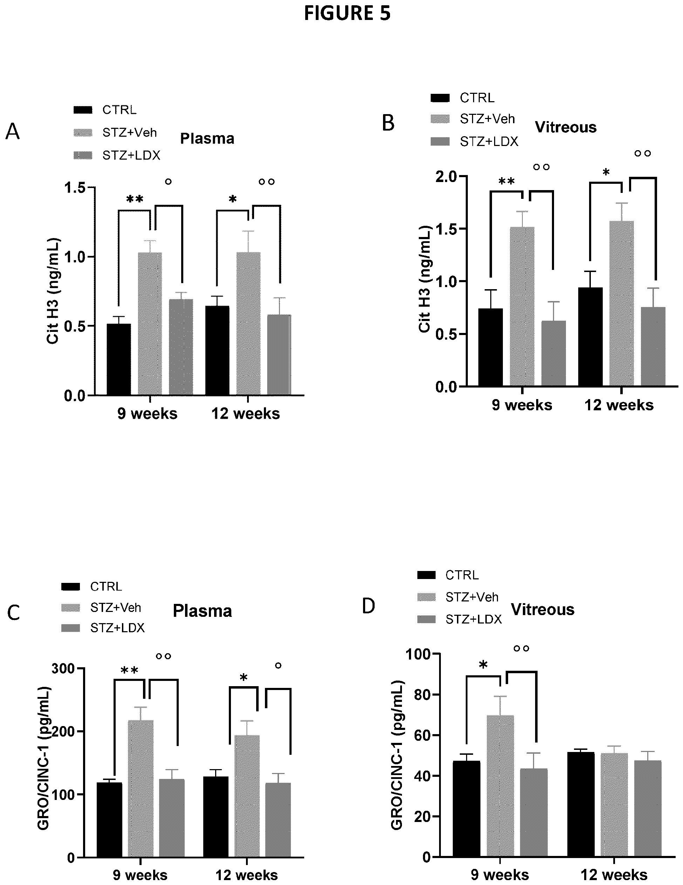

**Figure 5** shows graphs of Citrullinated Histone H3 (Cit H3) concentration (ng/mL) in plasma (A) and vitreous (B), and GRO/CINC-1 concentration (CXCL8) (pg/mL) in plasma (C) and vitreous (D), in CTRL, STZ+Veh and STZ+LDX animals (late treatment), according to Example 1. Data are represented as mean ± SEM (N= 4). Two-way ANOVA followed by Tukey's post hoc tests for multiple comparisons tests were performed among groups during the time course. The threshold of 0.05 was used for determining statistical significance. * and ° indicate significant differences vs CTRL and STZ+Veh, respectively.

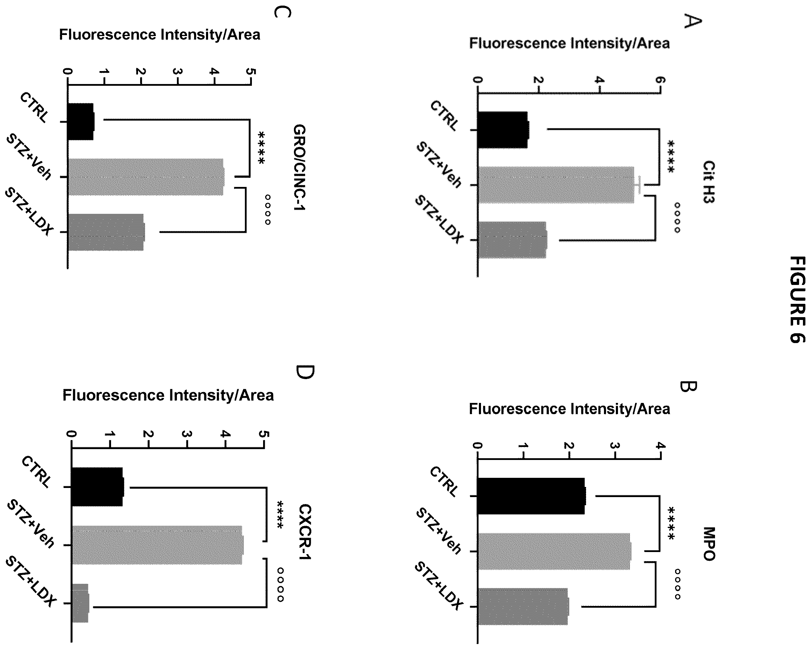

**Figure 6** shows graphs of (A) the relative quantification (fluorescence intensity per area) of Citrullinated Histone H3 (Cit H3) expression in the retina layers, (B) the relative quantification (fluorescence intensity per area) of MPO expression in the retina layers, (C) the relative quantification (fluorescence intensity per area) of GRO/CINC-1 expression in the retina layers, (D) the relative quantification (fluorescence intensity per area) of CXCR1 expression in the retina layers, of CTRL, STZ+Veh and STZ+LDX animals (late treatment). Data are represented as mean ± SEM (N=3). Two-way ANOVA followed by Tukey's post hoc tests for multiple comparisons tests were performed among groups during the time course. The threshold of 0.05 was used for determining statistical significance. * and ° indicate significant differences vs CTRL and STZ+Veh, respectively.

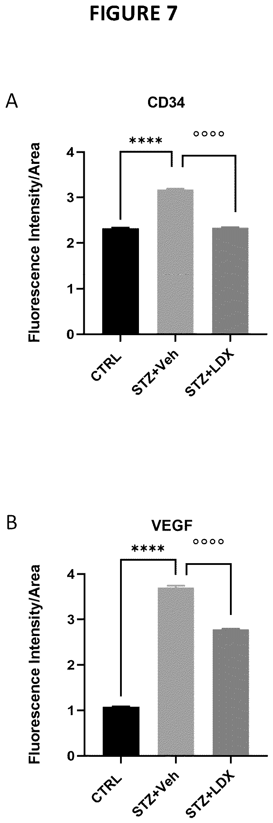

**Figure 7** shows graphs of (A) the relative quantification (fluorescence intensity per area) of CD34 expression in the retina layers, (B) the relative quantification (fluorescence intensity per area) of VEGF expression in the retina layers, of CTRL, STZ+Veh and STZ+LDX animals (late treatment). Data are represented as mean ± SEM (N=3). Two-way ANOVA followed by Tukey's post hoc tests for multiple comparisons tests were performed among groups during the time course. The threshold of 0.05 was used for determining statistical significance. * and ° indicate significant differences vs CTRL and STZ+Veh, respectively.

### DETAILED DESCRIPTION OF THE INVENTION

It has been surprisingly found that CXCL8 inhibitors are effective in the treatment of diabetes-associated comorbidities, in particular diabetic retinopathy and/or diabetic neuropathy.

The inventors have surprisingly found out that said CXCL8 inhibitors are particularly effective in treating painful diabetic peripheral neuropathy, by drastically reducing pain behaviours (i.e., mechanical allodynia and increased thermal threshold), as well as in treating diabetic retinopathy, in particular non-proliferative diabetic retinopathy, by effectively reducing NET-related inflammation biomarkers and VEGF level (which is one of the main contributor of the collapse of the inner blood retinal barrier when present in an excessive concentration), independently from the efficacy of said inhibitors in treating diabetes, in particular the metabolic profile thereof.

In fact, while said inhibitors are surprisingly effective in treating the above-mentioned diabetes-associated comorbidities in both the early treatment (i.e. the treatment initiated in an early stage of the diseases) and the late treatment (i.e. the treatment initiated in an advanced stage of the diseases), according to the Example provided below, said CXCL8 inhibitors are particularly effective in treating diabetes only in the early treatment. Therefore, the treatment of said diabetes-comorbidities by the administration of said CXCL8 inhibitors is unexpectedly independent and uncorrelated to the improvement and the treatment of biochemical/metabolic diabetic parameters of the treated subjects.

Accordingly, the invention is directed to a CXCL8 inhibitor for use in the treatment of diabetes-associated comorbidities selected from diabetic retinopathy and/or diabetic neuropathy.

Preferably, said diabetes is diabetes mellitus, more preferably diabetes mellitus type 1.

Preferably, said diabetes is diabetes mellitus, more preferably diabetes mellitus type 2.

Preferably said diabetic retinopathy is non-proliferative diabetic retinopathy. Preferably, said diabetic neuropathy is diabetic peripheral neuropathy, more preferably painful diabetic peripheral neuropathy.

Accordingly, in a preferred embodiment of the present invention, said CXCL8 inhibitor is for use in the treatment of diabetes-associated comorbidities selected from non-proliferative diabetic retinopathy and/or painful diabetic peripheral neuropathy.

The term "CXCL8 inhibitor" in accordance with the present invention means any compound able to inhibit the biological activity of CXCL8.

Methods for determining the inhibition of the biological activity of CXCL8 and for classifying a compound as "CXCL8 inhibitor" are known in the art and are described, for example, in Moriconi et al., J. Med. Chem. 2007, 50, 3984-4002, and in Brandolini et al., Scientific Reports (2019) 9:11729.

Preferably, a CXCL8 inhibitor according to the present invention is a CXCL8 receptor(s) inhibitor.

Preferably said CXCL8 receptor(s) inhibitor is a CXCR1- or a CXCR1/2-inhibitor, which inhibits the activity of CXCL8 mediated by the CXCR1 receptor or mediated by both the CXCR1 and CXCR2 receptors.

Said CXCL8 receptor(s) inhibitor preferably inhibits the binding of CXCL8 to the CXCR1 receptor (CXCR1 receptor inhibitor) or to both the CXCR1 and CXCR2 receptors (dual CXCR1 and CXCR2 receptor inhibitor), or prevents or blocks the intracellular signaling activated by the binding of CXCL8 to the CXCR1 receptor (CXCR1 receptor inhibitor) or to both the CXCR1 and CXCR2 receptors (dual CXCR1 and CXCR2 receptor inhibitor).

According to a preferred embodiment, the CXCL8 receptor(s) inhibitor is an antagonist of the CXCR1 receptor or an antagonist of both the CXCR1 and CXCR2 receptors.

According to another preferred embodiment, the CXCL8 receptor(s) inhibitor is an allosteric inhibitor or an orthosteric antagonist of the CXCR1 receptor or of both the CXCR1 and CXCR2 receptors.

Alternatively, the CXCL8 inhibitor preferably binds to CXCL8, thus preventing its binding to its receptors.

Said CXCL8 receptor(s) inhibitor is preferably able to inhibit in an in-vitro assay at least 60%, preferably at least 70%, more preferably at least 80%, even more preferably at least 90% of PMNs chemotaxis induced by 1 nM CXCL8 at a concentration equal or below 500 nM, preferably below 100 nM. More preferably, the CXCL8 receptor(s) inhibitor according to the invention has an IC₅₀ value towards the CXCR1 receptor in the low nanomolar range, preferably lower than 10 nanomolar, more preferably in the range 0.02-5 nanomolar.

According to a further preferred embodiment, said CXCL8 inhibitor is selected from small molecules, peptides and antibodies, more preferably it is a small molecule.

The term "small molecule" refers to an organic compound having a molecular weight of 900 Daltons or lower.

CXCL8 inhibitors, in particular CXCL8 receptor(s) inhibitors, as defined above, are well known in the art.

To date, several CXCL8 inhibitors, such as small molecules, peptides and antibodies, have been disclosed, many of which are currently undergoing clinical trials or are used in therapy (Jie Jack, Expert Opinion Ther. Patents, 2001, 11(12), Chao J. et al., Bioorganic & Medicinal Chemistry Letters 17, 2007, p. 3778-3783, Busch-Petersen J. Current Topics in Medicinal Chemistry, 2006, 6, p. 1345-135, Allegretti et al, Immunology Letters 2012, Vol. 145, p. 68-78, Sitaru et al., Internal and Emergency Medicine (2023) 18: 1647-1664).

Preferably, the CXCL8 inhibitor according to the invention is selected from the group or consisting of:
- the anti-CXCL8 antibodies ABCream, BMS-986253 and ABX-IL-8;
- RP-72, PAC-G-31-P, SCH-N,
- Navarixin, having formula: 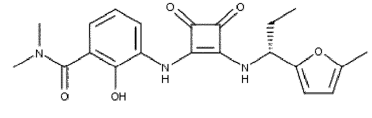
- SX-517, having formula: 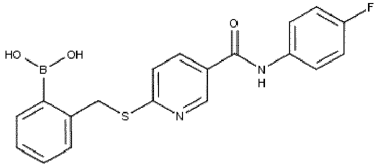
- SX-576, having formula: 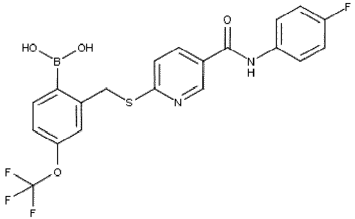
- SX-682 having formula: 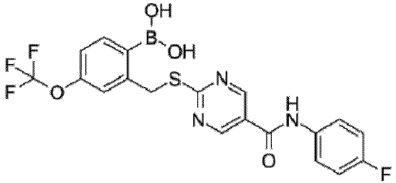
- compounds having formula: 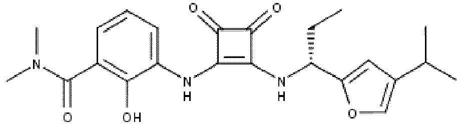 or 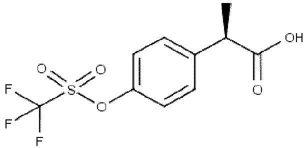 or 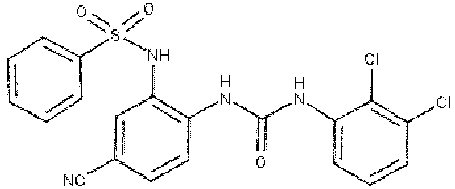 and
- 5-[3-(2-Fluorophenyl)ureido]-1-(2-hydroxypropyl)-1H-pyrazole-4-carboxylic acid ethyl ester
- 5-[3-(3-Fluorophenyl)ureido]-1-(2-hydroxypropyl)-1H-pyrazole-4-carboxylic acid ethyl ester
- 3-[2-[1(R)-(4-Bromofuran-2-yl)propylamino]-3,4-dioxo-1-cyclobutenylamino]-2-hydroxy-N,N-dimethylbenzamide
- 3-[2-[1(R)-(4-Chlorofuran-2-yl)propylamino]-3,4-dioxo-1-cyclobutenylamino]-2-hydroxy-N,N-dimethylbenzamide
- Trifluoromethanesulfonic acid 4-[1(R)-(N-isopropylcarbamoyl)ethyl]phenyl ester
- 2-Hydroxy-3-[4-[1(R)-(4-isopropylfuran-2-yl)propylamino]-1-oxo-1,2,5-thiadiazol-3-ylamino]-N,N-dimethylbenzamide
- 3-(2-Chlorophenylamino)-7-nitro-4H-1,2,4-benzothiadiazin-5-ol 1,1-dioxide
- 1-[3-[4-[3-(4-Fluorophenyl)isoxazol-5-yl]phenoxy]propyl]-4-methylpiperazine
- N-(2-[(2,3-Difluorobenzyl)sulfanyl]-6-[[(2R,3S)-3,4-dihydroxybutan-2-yl]oxy]pyrimidin-4-yl)azetidine-1-sulfonamide; and
- the compounds of formula (I) and (II) described hereinbelow.

According to one preferred embodiment, said CXCL8 inhibitor has general formula (I): 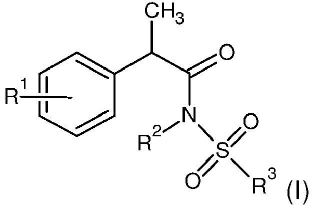 wherein
R¹ is selected from a linear or branched C₁-C₆ alkyl, benzoyl, phenoxy, and trifluoromethanesulfonyloxy;
R² is selected from hydrogen and a linear or branched C₁-C₃ alkyl; and
R³ is a linear or branched C₁-C₆ alkyl or trifluoromethyl,
or a pharmaceutically acceptable salt thereof.

According to the present invention, "C₁-C₆-alkyl" represents a linear or branched alkyl chain containing 1 to 6 carbon atoms.

R¹ is preferably selected from benzoyl, isobutyl, and trifluoromethanesulfonyloxy. R¹ is preferably linked to the phenyl ring in 3- or 4-position. According to the most preferred embodiment, R¹ is 3-benzoyl, 4-isobutyl or 4-trifluoromethanesulfonyloxy.

R² is preferably selected from hydrogen or methyl.

R³ is preferably selected from linear or branched C₁-C₆ alkyl, more preferably from linear of branched C₁-C₃ alkyl. According to the most preferred embodiment, R³ is methyl.

The chiral carbon of the compounds of formula (I) is in the RS or R configuration, more preferably it is in the R configuration.

Particularly preferred CXCL8 inhibitors compounds of formula (I) according to the invention are selected from:
- 2-(4-isobutylphenyl)propionyl methansulfonamide, preferably R-(-)-2-(4-isobutylphenyl)propionyl methansulfonamide (also known as reparixin) and pharmaceutically acceptable salts thereof, preferably the lysine salt thereof, and
- 2-(4-trifluoromethanesulfonyloxy)phenyl]-N-methanesulfonyl propionamide, preferably R(-)-2-(4-trifluoromethanesulfonyloxy)phenyl]-N-methanesulfonyl propionamide and pharmaceutically acceptable salts thereof, in particular the sodium salt thereof (also known as ladarixin or DF2156A).

Compounds of formula (I) are described in WO2000/024710A1 and WO2005/090295A2, which also disclose their method of synthesis. According to one preferred embodiment, said CXCL8 inhibitor has general formula (II): 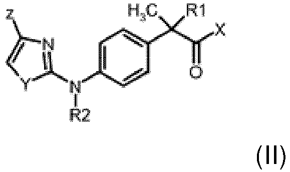 wherein:
R1 is hydrogen or linear or branched C₁-C₄ alkyl, more preferably methyl;
X is OH;
R2 is hydrogen or linear C₁-C₄ alkyl,
Y is a heteroatom selected from S, O and N,
Z is selected from linear or branched C₁-C₄ alkyl, linear or branched C₁-C₄ alkoxy, halo C₁-C₃ alkyl and halo C₁-C₃ alkoxy,
or a pharmaceutically acceptable salt thereof.

Preferably, the chiral carbon of the compounds of formula (II) is in the R or S configuration, more preferably in the S configuration.

Particularly preferred compounds of formula (II) according to the invention are 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl] amino} phenyl) propanoic acid or the sodium salt thereof, and 2-methyl-2-(4-{[4-(trifluoromethyl)-1,3-thiazol-2-yl]amino} phenyl)propanoic acid (DF2726Y) and pharmaceutically acceptable salts thereof, preferably the sodium salt (DF2726A).

Compounds of formula (II) are described in WO2010/031835A2, which also discloses their method of synthesis.

A particularly preferred CXCL8 inhibitor according to the invention is ladarixin.

The CXCL8 inhibitor for use according to the present invention may form stable pharmaceutically acceptable salts with a pharmaceutically acceptable organic or inorganic acid or base, and in such cases administration of a compound as a salt may be appropriate.

Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate. Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others. Non-toxic physiologically-acceptable salts are preferred, although other salts may be useful, such as in isolating or purifying the product.

The salts may be formed by conventional means, such as by reacting the free form of the product with one or more equivalents of the appropriate acid or base in a solvent or medium in which the salt is insoluble, such as for example water or ethanol, which is removed under vacuum or by freeze drying.

The present invention also includes the prodrugs, stereoisomers, isotope-labelled, for example deuterated, derivatives and enantiomers of the CXCL8 inhibitor compounds described above.

As used herein the term "prodrug" refers to an agent, which is converted into the parent drug in vivo by some physiological process (e.g., a prodrug on being brought to the physiological pH is converted to the desired drug form). Prodrugs are often useful because, in some situations, they may be easier to administer than the parent drug. They may, for instance, be bioavailable by oral administration whereas the parent drug is not. The prodrug may also have improved solubility in pharmacological compositions over the parent drug. An example, without limitation, of a prodrug would be a compound of the present invention wherein it is administered as an ester (the "prodrug") to facilitate the transfer across a cell membrane, where water solubility is detrimental, but then it is metabolically hydrolysed once inside the cell where water solubility is beneficial.

Prodrugs have many useful properties. For example, a prodrug may be more water-soluble than the ultimate drug, thereby facilitating intravenous administration of the drug. A prodrug may also have a higher level of oral bioavailability than the ultimate drug. After administration, the prodrug is enzymatically or chemically cleaved to deliver the ultimate drug in the blood or tissue.

Ester prodrugs of the CXCL8 inhibitor compounds disclosed herein are specifically contemplated. While not intending to be limiting, an ester may be an alkyl ester, an aryl ester, or a heteroaryl ester. The term alkyl has the meaning generally understood by those skilled in the art and refers to linear, branched, or cyclic alkyl moieties. C1-6 alkyl esters are particularly useful, where alkyl part of the ester has from 1 to 6 carbon atoms and includes, but is not limited to, methyl, ethyl, propyl, isopropyl, n-butyl, sec-butyl, iso-butyl, t-butyl, pentyl isomers, hexyl isomers, cyclopropyl, cyclobutyl, cyclopentyl, cyclohexyl, and combinations thereof.

Certain CXCL8 inhibitors may exist in tautomeric forms, and this invention includes all such tautomeric forms of those compounds unless otherwise specified.

Unless otherwise stated, structures depicted herein are also meant to include all stereochemical forms of the structure; i.e., the R and S configurations for each asymmetric centre. Thus, single stereochemical isomers as well as enantiomeric and diastereomeric mixtures of the present compounds are within the scope of the invention. Thus, this invention encompasses each diastereomer or enantiomer substantially free of other isomers (>90%, and preferably >95%, free from other stereoisomers on a molar basis) as well as a mixture of such isomers in any ratio.

Particular optical isomers can be obtained by resolution of the racemic mixtures according to conventional processes, e.g., by formation of diastereomeric salts, by treatment with an optically active acid or base or enzymatically. Examples of appropriate acids are tartaric, diacetyltartaric, dibenzoyltartaric, ditoluoyltartaric, and camphorsulfonic acid and then separation of the mixture of diastereomers by crystallization followed by liberation of the optically active bases from these salts. A different process for separation of optical isomers involves the use of a chiral chromatography column optimally chosen to maximize the separation of the enantiomers. Still another method involves synthesis of covalent diastereomers by reacting compounds of the invention with an optically pure acid in an activated form or an optically pure isocyanate. The synthesized diastereomers can be separated by conventional means such as chromatography, distillation, crystallization or sublimation, and then hydrolyzed to deliver the enantiomerically pure compound. Optically active compounds of the invention can be obtained by using active starting materials. These isomers may be in the form of a free acid, a free base, an ester or a salt.

The CXCL8 inhibitors for use according to the present invention may be in amorphous or crystalline form, including any polymorphic form.

According to a preferred embodiment, the CXCL8 inhibitor for use according to the present invention is administered orally.

According to another preferred embodiment, the CXCL8 inhibitor for use according to the present invention is administered by parenteral route, e.g. by intravenous, intraperitoneal, intracerebral, intrathecal, intracranial, intramuscular, intraarticular, intrasynovial, intrasternal, intraocular, intraarterial, subcutaneous, intracutaneous or intralesional injection or infusion techniques.

According to a further preferred embodiment, the CXCL8 inhibitor for use according to the present invention is administered by a route of administration selected from topical, buccal and suppository.

According to a further preferred embodiment, the CXCL8 inhibitor for use according to the present invention is administered by sustained release systems.

According to the further preferred embodiment, in which the CXCL8 inhibitor is for use in treating diabetic retinopathy, in particular non-proliferative diabetic retinopathy, according to the above, said CXCL8 inhibitor is administered intravitreally.

The invention is also directed to a pharmaceutical composition comprising at least one CXCL8 inhibitor and at least one pharmaceutically acceptable excipient or carrier, for use in preventing and/or treating diabetes-associated comorbidities selected from diabetic retinopathy and/or diabetic neuropathy, according to the above.

Preferably, said at least one CXCL8 inhibitor is selected from the CXCL8 inhibitors disclosed above.

Preferably, said pharmaceutically acceptable excipient or carrier as used herein includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions.

Moreover, the composition of the present invention may be formulated into inhalable or injectable dosage forms such as solutions, suspensions, and emulsions by further adding diluents, dispersants, and surfactants.

The composition of the present invention may also be formulated into dosage forms suitable for the intravitreal administration, in particular for the treatment of diabetic retinopathy, preferably non-proliferative diabetic retinopathy.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any particular limitation, any material suitable for preparing a pharmaceutical composition to be administered to a living being.

The dosage forms can also contain other traditional ingredients such as: preservatives, stabilizers, surfactants, buffers, osmotic regulators, emulsifiers, sweeteners, colorants, flavorings and the like.

Preferably, the pharmaceutical composition of the present invention is prepared in suitable dosage forms comprising an effective amount of a CXCL8 inhibitor, a pharmaceutically acceptable salt thereof, or a prodrug thereof, and at least one inert pharmaceutically acceptable excipient.

The administration of the pharmaceutical composition of the present invention to a patient is in accordance with known methods and may comprise from one to several oral administrations per day (for example, two times a day (BID) or four times a day (QID)), parenteral administration (including intravenous, intraperitoneal, intracerebral, intrathecal, intracranial, intramuscular, intraarticular, intrasynovial, intrasternal, intraocular, intraarterial, subcutaneous, intracutaneous or intralesional injection or infusion techniques), topical, buccal and suppository administration, or by sustained release systems among other routes of administration.

The amount of a CXCL8 inhibitor or the pharmaceutically acceptable salt thereof in the pharmaceutical composition of the present invention can vary over a wide range depending on known factors, for example, the molecule used, the severity of the disease, the patient's body weight, the dosage form, the chosen route of administration, and the number of administrations per day. However, a person skilled in the art can determine the optimum amount in easily and routinely manner.

The dosage forms of the pharmaceutical composition of the present invention can be prepared by techniques that are familiar to a pharmaceutical chemist, and comprise mixing, granulation, compression, dissolution, sterilization and the like.

According to a further embodiment, the invention is directed to a method of preventing and/or treating diabetes-associated comorbidities selected from diabetic retinopathy and/or diabetic neuropathy, as disclosed above, comprising administering an effective amount of said CXCL8 inhibitor or the pharmaceutically acceptable salt in a subject in need thereof.

An "effective amount" according to the present invention means an amount sufficient to achieve treatment of the disease. Determination of the effective amounts is well within the capability of those skilled in the art based upon the achievement of a desired effect. An effective amount will depend on factors including, but not limited to, the weight of a subject and/or the degree of the disease or unwanted condition from which a subject suffers.

According to a preferred embodiment, the invention relates also to the use of at least one CXCL8 inhibitor in the manufacture of a medicament for the treatment of diabetes-associated comorbidities selected from diabetic retinopathy and/or diabetic neuropathy, as disclosed above. Preferably, said at least one CXCL8 inhibitor is selected from the CXCL8 inhibitors disclosed above.

The term "treatment" as used herein refer to the eradication/amelioration of the disorder being treated or of one or more of the symptoms associated thereof, notwithstanding the fact that the patient may still be afflicted with the underlying disorder.

The invention is further illustrated by the following examples.

### EXPERIMENTAL SECTION

### Example 1

### STZ-induced type 1 diabetes animals and treatments.

Male Wistar rats (225-240 g) (Envigo, Italy) were housed 2-3 per cage under controlled illumination (12:12 h light:dark cycle; light on 06.00 h) and standard environmental conditions (room temperature 22 ± 1 °C, humidity 60 ± 10%).

Following one week of adaptation, animals were divided in three groups: control animals (CTRL), Streptozotocin(STZ)-induced type 1 diabetes animals treated by vehicle solution (STZ+Veh), and Streptozotocin(STZ)-induced type 1 animals treated by Ladarixin (STZ+LDX), according to the below-described treatments.

The control animals (CTRL) received a single intraperitoneal injection of a 0.1M citrate buffer solution.

Experimental T1D was induced by a single intraperitoneal injection of Streptozotocin (STZ) at a dose of 65 mg/kg in 0.1M cold citrate buffer (according to Long L. et al., Hyperglycemia induced testicular damage in type 2 diabetes mellitus rats exhibiting microcirculation impairments associated with vascular endothelial growth factor decreased via PI3K/Akt pathway. Oncotarget. 2018 Jan 4;9(4):5321-5336). They were allowed to drink a 10% dextrose solution overnight and then placed back on standard lab chow with water ad libitum. Blood glucose levels were measured one-week later STZ injection and all animals with levels below 300 mg/dl were excluded from the further evaluations.

Besides diabetes, STZ also induced diabetes-associated comorbidities, comprising non-proliferative diabetic retinopathy, and painful diabetic peripheral neuropathy (PDPN).

In vivo evaluations (blood glucose, mechanical allodynia and thermal nociceptive threshold) were measured at baseline (day 0) and once per week until the end of experimental observation.

The thus-obtained STZ-induced diabetic rats received a daily intra gastric (i.g.) treatment with a vehicle solution (STZ+Veh), or a Ladarixin solution (STZ+LDX), from 4 to 8-weeks post-STZ (early treatment) or from 8 to 12 weeks post-STZ (late treatment).

The above-mentioned vehicle solution consisted of sterile water.

The above-mentioned Ladarixin solution consisted of Ladarixin (15 mg/Kg) dissolved in sterile water.

In early-treated groups, plasma and vitreous samples were collected at 3, 5 and 7 weeks post-STZ, whereas dorsal root ganglia (DRG) and sciatic nerve were collected at the end of in vivo evaluations (8 weeks post-STZ).

In late-treated animals, blood samples were collected every 2 weeks from the induction of diabetes, whereas vitreous, eyes or pancreas were collected at 9 and 12 weeks post-STZ and, depending on the test according to the below, at 8 weeks post-STZ, too.

### Statistical analysis

### Data analysis

Data were analyzed using GraphPad Prism version 9.4.0 (GraphPad Software). Data from all in vivo experiments are expressed as mean ± SEM. Two-way ANOVA followed by Tukey's post hoc tests was used to analyze differences between groups, using treatment and testing time as factors in the analysis. One-way ANOVA followed by Dunnett post hoc comparisons was used to assess differences within groups (post-drug vs pre-drug) for behavioral evaluations. One-way ANOVA followed by Dunnett post hoc comparisons was used to assess significant differences between groups for ex vivo biochemical evaluations. The level of significance was set at p< 0.05.

### - Effects of early treatment with Ladarixin on blood glucose levels in STZ-induced diabetic rats.

Glucose blood level measurements were assessed before STZ administration (day 0), and once per week until the end of experimental observations.

Measurements of glucose concentration were obtained from whole blood samples taken from rat tail vein (the parameter expressed in milligrams per deciliter was determined by use of a standard clinical blood glucometer set for testing glycemia (GlucoMen LX2; A. Menarini Diagnostics, Winnersh, United Kingdom).

The results of Glucose blood level measurement are reported in Figure 1A. On the day before STZ injection, the basal blood glucose levels were measured in all animals with a mean value of 124.71 ± 1.03 mg/dL. Significant increase of average plasma glucose levels to 232.46 ± 7.0 mg/dL was observed in all STZ rats, starting from 2 weeks after STZ injection, as compared to CTRL group (122.8 ± 2.3 mg/dL; p<0.0001) that kept stable during the entire duration of experiments.

LDX early treatment (i.e., from 4 to 8 weeks post-STZ) showed a partial reduction of the hyperglycemic state in STZ-LDX rats from 1-week post-drug (6 weeks post-STZ) (425.2 ± 10.4 mg/dL; p<0.0001) up to the end of observations (8 weeks post-STZ) (414.12 mg/dL; p<0.0001) compared with STZ+Veh rats, that instead did not show any change in blood glucose levels until the end of experiment (562 ± 10.2 mg/dL).

### - Effects of early treatment with LDX on AcAc and BHB ketone bodies in STZ-induced diabetic rats.

Plasma concentrations of acetoacetate (AcAc) and β-hydroxybutyrate (BHB) ketone bodies were determined at 3, 5 and 7 weeks post-STZ, by an enzymatic colorimetric method using a commercially available kit (Elabscience Biotechnology Inc. Huston. Texas. USA or R&D Systems. Minneapolis. MN. USA), according to the protocol supplied by the manufacturer.

The results of the plasma concentration of AcAc and BHB are reported in Figure 1B and 1C, respectively.

The plasma ketone body concentration resulted significantly higher in STZ rats, starting from 3 weeks post-STZ (557.33 ± 22.9 µM; p=0.01) for AcAc, and 5 weeks post-STZ for BHB (709.23 ± 80.1 nM; p=0.045), as compared to CTRL rats (269.87 ± 23.3 µM; p=0.04 and 258.54 ± 21.1 nM).

Early treatment with LDX significantly reduced AcAc and BHB plasma levels in STZ+LDX rats, as compared to STZ+Veh rats (287.7 ± 22 µM; p=0.0018 and 246.7 ± 15.7 nM p=0.0006).

### - Effects of early treatment with LDX on plasma metabolic parameters in STZ-induced diabetic rats.

Plasma concentrations of insulin, glycated hemoglobin (HbA1c), triglycerides (TG), total cholesterol (TC), high density lipoprotein (HDL)-cholesterol, and low-density lipoprotein (LDL)-cholesterol were determined at 3, 5 and 7 weeks post-STZ, by an enzymatic colorimetric method using a commercially available kit (Elabscience Biotechnology Inc. Huston. Texas. USA or R&D Systems. Minneapolis. MN. USA), according to the protocol supplied by the manufacturer.

### -- Insulin

A significant decrease of insulin was found in STZ+Veh rat plasma, at 3, 5 and 7 weeks post-STZ (71 ± 17 pg/ml; p=0.05, 7 weeks post-STZ) as compared with CTRL rat plasma (125 ± 22 pg/ml, 7 weeks post-STZ); interestingly, LDX early treatment normalized insulin levels after 1 and 3 weeks of treatment in STZ+LDX rats (5 and 7 weeks post-STZ) (135 ± 13 pg/ml; p=0.025), as shown in Figure 1D.

### --Glycated hemoglobin (HbA1c)

A significant increase in the plasma level of HbA1c was observed in STZ+Veh rats at 5 and 7 weeks post-STZ (34 ± 6 ng/ml; p=0.03, 7 weeks post-STZ), as compared with CTRL rats (14 ± 1 ng/ml; 7 weeks post-STZ); instead, the LDX early treatment normalized plasma level of HbA1c in STZ+LDX rats (18 ± 3 ng/ml; p=0.008, 7 weeks post-STZ) as compared to STZ+Veh animals, as shown in Figure 1E.

### --Triglycerides

Measurement of plasma triglycerides (TG) levels indicated a strong increase in STZ+Veh rats at 5 and 7 weeks post-STZ (131± 10 mg/ml; p=0.017, 7 weeks post-STZ), compared to the CTRL group (62 ± 10 mg/ml; 7 weeks post-STZ); instead, the LDX early treatment significantly decreased the TG levels in STZ+LDX rats (71 ± 9 mg/ml, p=0.0006; 7 weeks post-STZ) in comparison with STZ+Veh rats, as shown in Figure 1F.

### -- Cholesterol (total, HDL, LDL)

Diabetes induction resulted in a significant increase of plasma total cholesterol in STZ+Veh rats (110 ± 8 mg/ml, p=0.0023; 7 weeks post-STZ) at 5 and 7 weeks post-STZ, compared to the CTRL group (74 ± 7 mg/ml, 7 weeks post-STZ), as shown in Figure 1G).

Moreover, a significant decrease of HDL-cholesterol (13 ± 2 mg/ml, p<0.0001; 7 weeks post-STZ), and an increase of LDL-cholesterol (60 ± 2 mg/ml, p<0.0001; 7 weeks post-STZ) in the plasma of STZ+Veh rats as compared to the CTRL group (48 ± 1 mg/ml and 21 ± 4 mg/ml, 7 weeks post-STZ, respectively) was found, as shown in Figures 1H and 1I, respectively). The LDX early treatment significantly normalized the levels of total cholesterol (87 ± 8 mg/ml, p=0.049; 7 weeks post-STZ), HDL- (33 ± 4 mg/ml, p=0.0001; 7 weeks post-STZ) and LDL-cholesterol (37 ± 4 mg/ml, p=0.0001; 7 weeks post-STZ) in STZ+LDX, as compared with STZ+Veh rats (as shown in Figures 1G, 1H and 1I, respectively).

### - Effects of early treatment with LDX on mechanical allodynia, due to painful diabetic peripheral neuropathy, in STZ-induced diabetic rats.

Mechanical allodynia was assessed by determining paw withdrawal response, measured as "paw withdrawal threshold" (PWT), using the up-down method (i.e. Von Frey test), according to Chaplan SR, et al., Quantitative assessment of tactile allodynia in the rat paw. Journal of Neuroscience Methods. 1994;53(1):55-63.

All animals were allowed to acclimate for ~30-45 minutes on an elevated mesh platform in an enclosure (Ugo Basile, Italy).

Calibrated von Frey filaments for rats (Stoelting, Wood Dale, IL, ranging from 4 g to 100 g bending force) were applied to the midplantar surface of the hind paw for 3-4 s (Messaoudi I. et al., Unilateral 6-Hydroxydopamine-Lesioned Rat as Relevant Model to Study the Pain Related to Parkinson's Disease. NNB. 2020:1-5). The threshold was captured as the lowest force (g) that evoked scratching or licking of the stimulated hind-paw.

Animals were tested at baseline (d 0) before the STZ injection and weekly until 12 weeks post-STZ.

On the day before STZ injection, the basal withdrawal threshold values were recorded with von Frey test.

Rats were assigned to experimental groups randomly to minimize the differences among group averages (81.2 ± 3.07 g).

Figure 2A shows the results of mechanical allodynia test, determined by said von Frey test.

In particular, the development of mechanical allodynia in STZ+Veh rats was observed starting from 2 weeks after diabetes induction with a significant peak at 5 weeks post-STZ (18.11 ± 3.86 g, p<0.0001), as compared to the CTRL group (95 ± 5 g), accordingly with increased blood glucose levels (Fig. 1A), and the reduced mechanical threshold was reverted in STZ animals treated with LDX (early treatment).

More specifically, this effect started 1 week from the beginning of the treatment (5 weeks post-STZ), and it lasted until 4 weeks post-treatment. In fact, at peak-time of anti-allodynic effect (2 weeks post-drug), the mean paw threshold in STZ+LDX rats (68.55 ± 7.03 g) was very close to that observed in CTRL rats (95 ± 5 g).

Mixed-effects model analysis showed a significant effect of treatments (F2, 35 = 105.35, P<0.0001), of time (F5, 175 = 15.23, P<0.0001), and significant interaction for factors time × treatment (F10, 175 = 7.77, P<0.0001) was observed.

### - Effects of early treatment with LDX on thermal nociceptive latency, due to painful diabetic peripheral neuropathy, in STZ-induced diabetic rats.

The thermal nociceptive latency was determined by the tail flick test, which involves application of a heat stimulus to the tail of animals and the time taken for the tail to "flick" or twitch is recorded (Fred E. D'amour et al., A method for determining loss of pain sensation, Journal of Pharmacology and Experimental Therapeutics,1941, 72 (1) 74-79).

The heat stimulus applied was radiant, and the nociceptive threshold was measured by a tail-flick apparatus (Harvard Apparatus, USA).

A heat stimulus was applied after 5 cm from the caudal tip of the tail. The reaction time between the onset of heat stimulus and the movement of tail was determined by an automatic sensor and recorded as tail-flick latency (TFL) and it was measured in seconds (s). The light source (heat radiant) was set at an intensity that yields baseline TFL values in the range of 7-8 s. If the animal failed to flick its tail within 15 s (cut-off point), the tail was removed from the coil to prevent tissue damage.

The results of the tail flick test are reported in Figure 2B.

Before STZ administration, rats showed a mean of tail flick latency from radiant heat of 7.02 ± 0.23 sec.

Starting from 2 weeks following STZ administration, the thermal pain threshold significantly decreased in STZ+Veh rats (4 ± 0.53 sec, p<0.05) as compared to CTRL animals (7.25 ± 0.7 sec); however, LDX early treatment significantly increased the tail flick latency with highest effect 1-week post-drug (9.03 ± 0.39 sec) in STZ+LDX, as compared to STZ+Veh rats (p<0.0001), which in fact showed a reduced tail flick latency until the end of treatment (4.7 ± 0.24 sec).

Mixed-effects model analysis showed a significant effect of treatments (F2,35 = 42.18, P<0.0001), of time (F5, 175 = 4.36, P=0.0031), and significant interaction for factors time × treatment (F10, 175 = 6.49, P<0.0001) was observed.

### - Effects of early treatment with LDX on inflammatory mediators, citrullinated histone H3 and GRO/CINC-1, in plasma and vitreous in STZ-induced diabetic rats.

The levels of citrullinated histone H3 and GRO/CINC-1 were evaluated in the STZ-induced diabetic rats at 3, 5 and 7 weeks post-STZ, by enzymatic colorimetric methods (ELISA) using commercially available kits. The assay was performed according to the manufacturer's instructions (Elabscience Biotechnology Inc. Huston, Texas, USA or R&D Systems Minneapolis, MN, USA).

A significant increase of citrullinated Histone H3 (Cit H3) was detected in the plasma and vitreous of STZ+Veh rats at 3, 5 and 7 weeks post-STZ, as compared with CTRL rats; instead, LDX early treatment significantly reduced these effects in plasma and vitreous of STZ+LDX rats, as compared with STZ+Veh rats at 7 weeks post-STZ (3 weeks post-drug), as shown in Figures 3A and 3B, respectively.

Likewise, increased levels of GRO/CINC-1 in plasma and vitreous of STZ+Veh rats was observed at 5 and 7 weeks post-STZ, and these effects were significantly reverted by LDX early treatment at 7 weeks post-STZ (1- and 3-weeks post-drug) in STZ+LDX rats, as shown in Figures 3C and 3D, respectively.

### - LDX late treatment is ineffective in mitigating STZ-induced diabetes and associated inflammatory/metabolic parameters.

The following inflammatory and metabolic T1D parameters were assessed to evaluate the efficacy of the LDX late treatment T1D and the related conditions.

### -- Glucose level

Blood glucose levels were determined according to the above, and the results are shown in Figure 4A.

On the day before STZ injection, the basal blood glucose levels were measured in all animals with a mean value of 90.6 ± 2.89 mg/dL.

Significant increase of average plasma glucose levels to 540.6 ± 24.41 mg/dL was observed in all STZ rats, starting from 2 weeks after STZ injection, as compared to CTRL group (89.1±3.21 mg/dL; p<0.0001) that kept stable during the entire duration of experiments.

Surprisingly, LDX late treatment (i.e., from 8 up to 12 weeks post-STZ) was not able to reduce the hyperglycemic state in STZ+LDX rats (550.4 ± 23.04 mg/dL; p=0.73 at 9 weeks post-STZ) as compared to STZ+Veh rats (574.6 ± 22.06 mg/dL).

### -- Glucagon and insulin in pancreatic islets

Glucagon and insulin contained in the pancreatic islet of the tested rats were determined by immunofluorescence, according to the protocol reported above and applied to pancreas sections.

In particular, pancreases were harvested by the perfused PBS and paraformaldehyde rats, as disclosed above, and the pancreases were then postfixed for 4h in the perfusion fixative, cryoprotected for 72h in 30% sucrose in 0.1M phosphate buffer and frozen in O.C.T. embedding compound. The tissue sections (10µm) were cut, and thaw mounted onto glass slides as disclosed above.

The pancreas slides were incubated overnight with primary antibody specific for Insulin (rabbit Alexa Fluor^{®} 647 Anti-Insulin antibody; 1:300; Abcam) and Glucagon (mouse Anti-Glucagon antibody; 1:500; Abcam).

Following incubation sections were washed 3 times and incubated with the secondary Alexa Fluor^{®} conjugated antibodies (1:1000; Invitrogen).

Slides were treated according to the above, and the images were analyzed using Image-J as previously described.

The immunofluorescence analysis results shown in Figure 4B revealed a decrease in insulin positive staining ("Ins⁺ cells per islet") in STZ group compared to CTRL rats (40,17 ± 11,42 vs 278,5 ± 63,44; p=0,001), suggesting a reduction of β- cells number, and that 1 week of LDX late treatment was not effective in insulin labelling as compared with STZ+Veh group (82,0 ± 8,56 vs 40,17 ± 11,42; p= 0,71).

Similarly, the glucagon staining ("Glucagon⁺ cell per islet") results shown in Figure 4C revealed a mild α-cell damage induced by STZ compared to the CTRL group (64,83 ± 13,93 vs 124,0 ± 19,56; p=0,051), and no significant differences in the glucagon labelling between STZ+Veh and STZ+LDX (late treatment) rats were detected (64,83 ± 13,93 vs 75,50 ± 14,58; p=0,888).

### -- Plasmatic insulin and C-peptide

In order to evaluate the pancreatic β-cell functionality, insulin and C-peptide levels were measured at 9 and 12 weeks post-STZ (1 and 3 weeks post-LDX), as well as at 8 weeks post-STZ ("pre-LDX", namely the day before the administration of Ladarixin).

Plasma concentrations of insulin and peptide-C were determined by enzymatic colorimetric methods using commercially available kits; the assay was performed according to the protocol supplied by the manufacturer the manufacturer's instructions (Elabscience Biotechnology Inc. Huston. Texas. USA or R&D Systems. Minneapolis. MN. USA).

A significant reduction of both insulin (107.88 ± 14.23 pg/ml, p<0.0001 at 9 weeks post-STZ) and C-peptide (300.44 ± 38.78 pg/ml, p<0.0001 at 9 weeks post-STZ) was observed in STZ rats at 9 and 12 weeks post-STZ as compared to CTRL group (257.45 ± 7.16 pg/ml Ins; 757.36 ± 12.86 pg/ml C-Peptide), as shown in Figures 4D and 4E, respectively.

Intriguingly, LDX late treatment did not modify the reduced levels of neither insulin (165.56 ± 12.39 pg/ml, p=0.08 at 9 weeks post-STZ) nor C-peptide (434.18 ± 27.95 pg/ml, p=0.095 at 9 weeks post-STZ) in STZ+LDX (Fig. 4D and E), as observed on blood glucose levels (Fig. 4A).

Moreover, HOMA-IR and HOMA-β were calculated based on the following formula: HOMA-IR= serum insulin (mmol/L)*(blood glucose (mmol/L)/22.5 and HOMA-β:[20 × fasting insulin (µIU/ml)]/[fasting glucose (mmol/ml) - 3.5]. HOMA-IR and HOMA-B confirmed the development of insulin resistance and insulin secretion dysfunction in STZ rats, and this alteration was not reverted by LDX late treatment in STZ+LDX rats, as shown Figures 4F and 4G, respectively.

### - LDX late treatment is surprisingly effective in treating specifically mechanical allodynia and thermal nociceptive latency, due to painful diabetic peripheral neuropathy, in STZ-induced diabetic rats.

Mechanical allodynia was determined according to the above-mentioned Von Frey test, and the thermal nociceptive latency was determined by the above-mentioned tail flick test.

The results of Von Frey test are shown in Figure 4H, and the tail flick test in Figure 4I.

On the day before STZ injection, the basal withdrawal threshold values were recorded with von Frey test (83.33 ± 5.38 g) and tail flick test (9.4 ± 0.25 s) in all groups of animals.

Significant reduction of mechanical (39.2± 11.28 g, p=0.0085) and thermal threshold (6.9± 0.74 s, p=0.02) was observed in STZ rats starting from 2 weeks after diabetes induction as compared to CTRL group (84± 4.89 g and 9.49± 0.36 s); surprisingly, the LDX late treatment showed a trend in increase since 1-week post-treatment, and its effect resulted significant at three weeks post-LDX on both Von Frey test (66.8± 8.38 g, p=0.018) and tail flick test (7.34 ± 0.88 s, p=0.021) in STZ+LDX rats, as compared to STZ+Veh rats.

### - Effects of late treatment with LDX on inflammatory mediators, citrullinated histone H3 and GRO/CINC-1, in plasma and vitreous in STZ-induced diabetic rats.

The levels of citrullinated histone H3 and GRO/CINC-1 (CXCL8) were measured in STZ-induced diabetic rats at 9 and 12 weeks post-STZ, by immunofluorescence according to the following protocol.

Similarly to previous experimental observations (Fig. 3A-D), a significant increase of citrullinated histone H3 (Cit H3) was detected in plasma (1 ± 0.08 ng/ml, p=0.0019 at 9 weeks post-STZ) and vitreous (1.51 ± 0.15 ng/ml, p=0.0089 at 9 weeks post-STZ) of STZ+Veh rats at 9 and 12 weeks post-STZ, as compared with CTRL rats (0.51 ± 0.05 ng/ml, in plasma and 0.73 ± 0.18 ng/ml at 9 weeks post-STZ), as shown in Figures 5A and 5B, respectively.

Interestingly, LDX late treatment significantly reduced this increase in both plasma (0.69 ± 0.05 ng/ml, p=0.047 at 9 weeks post-STZ) and vitreous (0.62 ± 0.17 ng/ml, p=0.0027 at 9 weeks post-STZ) in STZ+LDX rats, as compared with STZ+Veh rats at 9 and 12 weeks post-STZ (1- and 3-weeks post-drug) (Fig. 5A and B, respectively).

Moreover, increased levels of GRO/CINC-1 at 9 weeks post-STZ in both plasma and vitreous (217± 20.63 pg/ml, p=0.0014 and 69.72 ± 9.38 pg/ml, p=0.024, respectively) of STZ+Veh rats were found, as compared to CTRL animals (118.86± 4.93 pg/ml and 47.13± 3.53 pg/ml) , and a similar increase was observed in plasma of STZ+Veh rats at 12 weeks post-STZ (176.86± 30.08 pg/ml, p=0.02) (Figures 5C and D).

Intriguingly, LDX late treatment restored basal levels of GRO/CINC-1 at 9 weeks post-STZ in both plasma and vitreous (124.06 ± 15.17 pg/ml, p=0.0009 and 43.5± 7.65 pg/ml, p=0.008) of STZ+LDX rats, and at 12 weeks post-STZ in plasma of STZ+LDX rats (118.07 ± 15.22 pg/ml, p=0.007) (Fig. 5 C and D).

### - Effect of late treatment with LDX on the GRO/CINC-1-CXCR1/2 pathway in the retina of STZ-induced diabetic rats.

The retinal expression of GRO/CINC-1 (CXCL8), CXCR1, citrullinated Histone H3 (Cit H3), and MPO in the STZ-induced diabetic rats was assessed by immunofluorescence analysis carried out on the eye sections according to the above immunofluorescence protocol.

In particular, primary antibody specific for Histone H3 (rabbit Anti-Histone H3 antibody; 1:200; Abcam), Myeloperoxidase (MPO) (mouse Anti-Myeloperoxidase antibody; 1:50; Abcam), GRO/CINC-1 (mouse IL-8/CXCL8 Monoclonal Antibody; 1:400; Thermo Fisher Scientific), CXCR1 (rabbit CXCR1 Polyclonal Antibody; 1:100; Elabscience), were used. Immunofluorescence analysis revealed an increased immunoreactivity of GRO/CINC-1 and CXCR1 in the retina of STZ+Veh rats as compared to CTRL animals, as shown in Figures 6A-6D.

In particular, 9 weeks after diabetes induction, an increased expression of GRO/CINC-1 (4,246 ± 0.0150 vs 0.7040 ± 0.0050; p<0.0001, Fig. 6C), as well as CXCR1 (4.434 ± 0.02135 vs 1.328 ± 0.01655; p<0.0001, Fig. 6D), Citrullinated Histone H3 (Cit H3) (5.142 ± 0.1689 vs 1.636 ± 0.02981; p<0.0001, Fig. 6A) and myeloperoxidase (MPO) (3.336 ± 0.01030 vs 2.334 ± 0.01435; p<0.0001, Fig. 6B), biomarkers of NETs formation, were detected in the STZ+Veh group as compared to the control rats.

Instead, LDX late treatment significantly reduced the fluorescence intensity in STZ+LDX rats for GRO/CINC-1 (2,070 ± 0,01789 vs 4,246 ± 0,0150; p<0,0001), CXCR1 (0,4300 ± 0,009487 vs 4,434 ± 0,02135; p<0,0001), Cit H3 (2,242 ± 0,01393 vs 5,142 ± 0,1689; p<0,0001) and MPO (1.972 ± 0.008602 vs 3.336 ± 0.01030; p<0.0001), as shown said Figures.

### - Effect of late treatment with LDX on the expression of VEGF and CD34 in the retina of STZ-induced diabetic rats.

The expression of VEGF and CD34, a marker of cell proliferation in the retina of STZ-induced diabetic rats, was investigated by immunofluorescence analysis carried out on the eye sections according to the above immunofluorescence protocol.

In particular, primary antibody specific for VEGF (rabbit VEGFD Recombinant Rabbit Monoclonal Antibody; 1:200; Thermo Fisher Scientific) and CD34 (rabbit CD34 Polyclonal Antibody; 1:100; Elabscience) were used.

VEGF expression was located in retinal ganglion cell layer and the outer plexiform layer (data not shown), and the CD34 expression was located in the vascular endothelial cells (data not shown).

9 weeks post diabetes induction, VEGF (2.784 ± 0.01364 vs 3.704 ± 0.04007; p<0,0001) and CD34 expressions were significantly (2.336 ± 0.01327 vs 3.174 ± 0.02015; p<0,0001) increased in the STZ+Veh rats' retinas than in the controls, as shown in Figures 7A and 7B, respectively; instead, the LDX late treatment significantly reduced both VEGF (2,784 ± 0,01364 vs 3,704 ± 0,04007; p ≤0,0001) and CD34 (2,336 ± 0,01327 vs 3,174 ± 0,02015; p ≤0,0001) in STZ+LDX rats compared to STZ+Veh rats.

## Claims

1. A CXCL8 inhibitor for use in the treatment of diabetes-associated comorbidities selected from diabetic retinopathy and/or diabetic neuropathy.

2. A CXCL8 inhibitor for use according to claim 1, wherein said diabetic retinopathy is non-proliferative diabetic retinopathy.

3. A CXCL8 inhibitor for use according to claim 1, wherein said diabetic neuropathy is diabetic peripheral neuropathy (DPN), preferably painful diabetic peripheral neuropathy (PDPN).

4. A CXCL8 inhibitor for use according to claims 1 to 3, wherein said inhibitor is selected from the group consisting of:
- the anti-CXCL-8 antibodies ABCream, BMS-986253 and ABX-IL-8;
- RP-72, PAC-G-31-P, SCH-N,
- Navarixin, having formula: 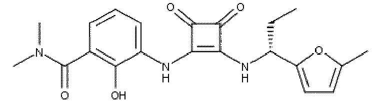
- SX-517, having formula: 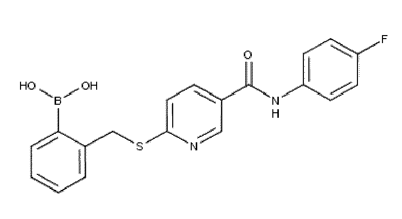
- SX-576, having formula: 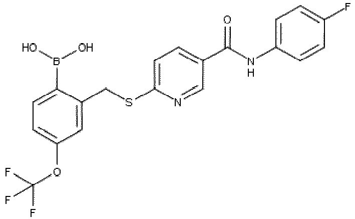
- SX-682 having formula: 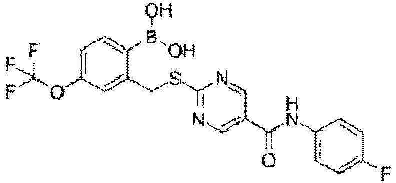
- compounds having formula: 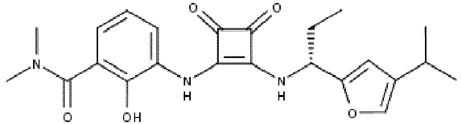 or 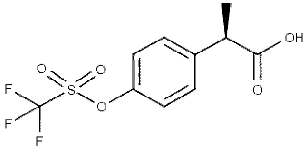 or 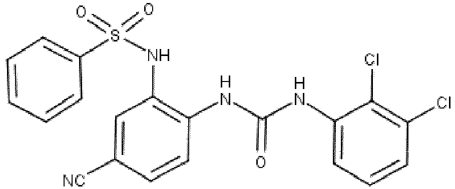 and
- 5-[3-(2-Fluorophenyl)ureido]-1-(2-hydroxypropyl)-1H-pyrazole-4-carboxylic acid ethyl ester
- 5-[3-(3-Fluorophenyl)ureido]-1-(2-hydroxypropyl)-1H-pyrazole-4-carboxylic acid ethyl ester
- 3-[2-[1(R)-(4-Bromofuran-2-yl)propylamino]-3,4-dioxo-1-cyclobutenylamino]-2-hydroxy-N,N-dimethylbenzamide
- 3-[2-[1(R)-(4-Chlorofuran-2-yl)propylamino]-3,4-dioxo-1-cyclobutenylamino]-2-hydroxy-N,N-dimethylbenzamide
- Trifluoromethanesulfonic acid 4-[1(R)-(N-isopropylcarbamoyl)ethyl]phenyl ester
- 2-Hydroxy-3-[4-[1(R)-(4-isopropylfuran-2-yl)propylamino]-1-oxo-1,2,5-thiadiazol-3-ylamino]-N,N-dimethylbenzamide
- 3-(2-Chlorophenylamino)-7-nitro-4H-1,2,4-benzothiadiazin-5-ol 1,1-dioxide
- 1-[3-[4-[3-(4-Fluorophenyl)isoxazol-5-yl]phenoxy]propyl]-4-methylpiperazine
- N-(2-[(2,3-Difluorobenzyl)sulfanyl]-6-[[(2R,3S)-3,4-dihydroxybutan-2-yl]oxy]pyrimidin-4-yl)azetidine-1-sulfonamide;
- a compound having general formula (I): 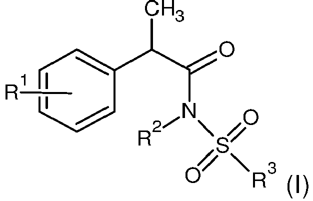 wherein
R¹ is selected from a linear or branched C₁-C₆ alkyl, benzoyl, phenoxy, and trifluoromethanesulfonyloxy;
R² is selected from hydrogen and a linear or branched C₁-C₃ alkyl; and
R³ is a linear or branched C₁-C₆ alkyl or trifluoromethyl,
or a pharmaceutically acceptable salt thereof; and
- a compound having general formula (II): 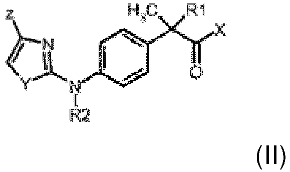 wherein:
R1 is hydrogen or linear or branched C₁-C₄ alkyl;
X is OH;
R2 is hydrogen or linear C₁-C₄ alkyl,
Y is a heteroatom selected from S, O and N,
Z is selected from linear or branched C₁-C₄ alkyl, linear or branched C₁-C₄ alkoxy, halo C₁-C₃ alkyl and halo C₁-C₃ alkoxy,
or pharmaceutically acceptable salts thereof.

5. A CXCL8 inhibitor for use according to claim 4, wherein the chiral carbon of the compounds of formula (I) is in the R configuration.

6. A CXCL8 inhibitor for use according to claim 4, wherein the chiral carbon of the compounds of formula (II) is in the S configuration.

7. A CXCL8 inhibitor for use according to claim 4, having general formula (I) which is selected from:
- 2-(4-isobutylphenyl)propionyl methansulfonamide, preferably R-(-)-2-(4-isobutylphenyl)propionyl methansulfonamide and pharmaceutically acceptable salts thereof, preferably the lysine salt thereof, and
- 2-(4-trifluoromethanesulfonyloxy)phenyl]-N-methanesulfonyl propionamide, preferably R(-)-2-(4-trifluoromethanesulfonyloxy)phenyl]-N-methanesulfonyl propionamide and pharmaceutically acceptable salts thereof, in particular the sodium salt thereof.

8. A CXCL8 inhibitor for use according to claim 4, having general formula (II) which is selected from 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl] amino} phenyl) propanoic acid or the sodium salt thereof and 2-methyl-2-(4-{[4-(trifluoromethyl)-1,3-thiazol-2-yl]amino} phenyl)propanoic acid and pharmaceutically acceptable salts thereof, preferably the sodium salt.

9. A CXCL8 inhibitor for use according to claim 4, which is 2-(4-trifluoromethanesulfonyloxy)phenyl]-N-methanesulfonyl propionamide, preferably R(-)-2-(4-trifluoromethanesulfonyloxy)phenyl]-N-methanesulfonyl propionamide or a pharmaceutically acceptable salt thereof, preferably the sodium salt thereof.
